# EUROPÄISCHE PATENTANMELDUNG

(11) **EP 0 634 190 A2**
(43) Veröffentlichungstag der Anmeldung: **18.01.1995**
(21) Anmeldenummer: 94110630.4
(22) Anmeldetag: 08.07.1994
(51) Int. Cl.: A61M 39/00, A61M 39/10

(54) **Verbindungsvorrichtung für medizinische Hohlraumleitungen**

(30) Priorität: 13.07.1993 DE 4323421
(71) Anmelder: Härle, Anton, Prof. Dr., D-48161 Münster (DE)
(72) Erfinder: Härle, Anton, Prof. Dr., D-48161 Münster (DE)

(57) **Zusammenfassung**

Die Erfindung bezieht sich auf eine Verbindungseinrichtung zum lösbaren Ankoppeln und Entkoppeln einzelner Komponenten, insbesondere medizinischer Leitungen, wobei eine, eine axiale Verschiebung der miteinander zu verbindenden Leitungsteile gegeneinander bewirkende Verbindungsvorrichtung vorgesehen ist, die die Verbindung oder Entkoppelung bewirkt und dabei gleichzeitig eine gasdichte Verbindung sicherstellt.

## Beschreibung

Die Erfindung bezieht sich auf eine Verbindungsvorrichtung für medizinische Hohlraum-Leitungssysteme.

Medizinische Hohlraum-Leitungssysteme werden für das Einleiten von Flüssigkeiten in einen Körper, vorzugsweise menschlichen Körper und auch für das Ableiten von Körpersekreten eingesetzt und bestehen üblicherweise aus mehreren Leitungsabschnitten, die mittels Verbindungseinrichtungen lösbar miteinander verbunden sind, wie dies beispielsweise sehr deutlich die US-PS 46 42 093 zeigt.

Als Verbindungseinrichtung werden in der Praxis ineinanderzuschiebende Steckverschlüsse eingesetzt, die aber keiner wesentlichen Zugbelastung widerstehen. Diese Verbindungseinrichtungen sind weiterhin nicht luftdicht.

Bei Leitungssystemen, bei denen die Abdichtung zwischen intra- und extraluminalem Raum von großer Wichtigkeit ist, werden daher in der Praxis Verbindungseinrichtungen aus elastischen Gummihülsen eingesetzt, die die einzelnen Leitungssegmente verkoppeln. Da z. B. die Wunddrainagen meist mit einer auf nahezu vollständiges Vakuum evakuierten Saugflasche als Aufnahmeeinheit, aber auch als Motor für die Saugfunktion durchgeführt werden, hat auch ein nur partieller Verlust des Unterdrucks in der Saugflasche oder dem Leitungssystem schwerwiegende Folgen. Insbesondere ist die vollständige Abdichtung der miteinander zu verbindenden Leitungsabschnitte in diesem medizinischen Bereich wichtig, da mit dem bloßen Auge der Unterdruck in der Saugflasche nicht überprüft werden kann und folglich durch ungenügende Abdichtung hervorgerufene Verluste des Unterdrucks in der Drainageleitung unentdeckt bleiben.

Die mittels elastischer Gummihülsen arbeitenden dichten Leitungssysteme bereiten bei der Lösung der einzelnen Leitungsabschnitte Schwierigkeiten. Das Lösen der einzelnen Leitungsabschnitte geschieht in üblicher Weise durch einen in Längsachse der Leitungsabschnitte wirkenden Zug. Dadurch entsteht durch die Vergrößerung des Innenraums ein zusätzlicher Unterdruck innerhalb des Leitungssystems. Bei der Diskonnexion tritt dann eine Implosion mit sofortiger Aerosolbildung auf und im nächsten Moment eine explosionsartige Versprühung dieser Aerosole in die Umgebung. Kam es zur Aerosolbildung mit einer aus dem Körper austretenden Flüssigkeit, die Krankheitserreger enthält, so ist auch das Aerosol damit beladen und kann so auf das ihm ausgesetzte Personal Infektionen übertragen.

Bei der heute aktuellen Hepatitis- und AIDS-Gefährdung des medizinischen Personals ist daher die bestehende Verbindungseinrichtung zwischen medizinischen Leitungsabschnitten nicht mehr zufriedenstellend.

Der Erfindung liegt die Aufgabe zugrunde, eine Verbindungseinrichtung für medizinische Leitungssysteme zu schaffen, die auch bei einer geringen Vorspannkraft, wie sie in aus dünnwandigen Kunststoffmaterialien geformten Gewindeabschnitten erreichbar ist, eine zuverlässige Abdichtung zwischen intra- und extraluminalem Raum erreichbar macht.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt wird vorgeschlagen, zum Verbinden der verschiedenen Leitungsabschnitte Drehverschlüsse einzusetzen. Die beiden miteinander zu verbindenden Leitungsenden sind dabei vorzugsweise mit Gewinde ausgerüstet, d. h. einem Außen- und einem Innengewinde, die miteinander zusammenwirken. Um einen luftdichten Abschluß zwischen Innen und Außen zu erreichen, sind gemäß einem wesentlichen Merkmal der Erfindung durch elastische oder mikroplastische Verformung abdichtende Kontaktbereiche vorgesehen, vorzugsweise aus gummielastischem Werkstoff bestehende Dichtmittel, die beim Zusammenschrauben der beiden Gewindebereiche zusammengepreßt werden und dabei die Abdichtung gewährleisten. Der Abdichtdruck soll dabei zwischen 1 und 10 N/mm² liegen.

Gemäß einem weiteren wesentlichen Merkmal der Erfindung wird auch ein Gewinde vorgeschlagen, das in sich selbst so ausgebildet ist, daß es bei seiner Verschraubung eine Abdichtung zwischen Innen und Außen gewährleistet.

Auch ist der Einsatz eines Bajonettverschlusses möglich.

Die Dichtmittel müssen dabei so ausgebildet sein, daß nach einem kurzen axialen Weg bereits eine vollständige Abdichtung auftritt, wobei vorzugsweise der Kontaktbereich im Gewinde mindestens 360^{o}, vorzugsweise aber 540^{o} oder 720^{o} beträgt. Um ein Überdrehen derartiger, üblicherweise aus Kunststoff bestehender Dichteinrichtungen zu verhindern, sind an der Außenseite der Verschlußvorrichtung Anzeigeeinrichtungen angebracht, die anzeigen, bei welcher Stellung der beiden gegeneinander zu verdrehenden Gewinde eine Abdichtung erreicht worden ist.

Schließlich beschäftigt sich die Erfindung damit, ein Hilfsmittel vorzuschlagen, das sicherstellt, daß ein ungewolltes Rückdrehen des Verschlusses entweder selbsttätig aufgrund der innerhalb des Leitungssystems herrschenden Torsion oder durch ein ungewolltes Betätigen durch den Patienten verhindert wird. Vorzugsweise wird zur Lösung dieses Problems eine abdichtend die Dichtverbindung übergreifende, elstische Hülse vorgeschlagen, die sich dicht an die Außenseite der beiden Leitungsabschnitte anlegt und dadurch das Verdrehen der beiden Leitungsabschnitte gegeneinander verhindert.

Ausführungsbeispiele der Erfindung werden nachfolgend anhand der Zeichnungen erläutert.

Die Zeichnungen zeigen dabei in
- Fig. 1: schaubildlich die Anordnung einer Saugflasche mit einer Verbindungsleitung, in
- Fig. 2: in wesentlich größerem Maßstab eine erste Ausführungsform der Verbindungsvorrichtung, in
- Fig. 3: eine weitere Ausführungsform der Verbindungsvorrichtung, in
- Fig. 4: eine wiederum abgeänderte Ausführungsform der Verbindungsvorrichtung, in
- Fig. 5: die Anordnung eines Arretierungsmittels, um die Verbindungsvorrichtung in ihrer Schließstellung zu arretieren, in
- Fig. 6: eine abgeänderte Ausführungsform der Abdichtvorrichtung, in
- Fig. 7: eine konische Aufnahme zur Unterstützung oder zur Erreichung der Abdichtung und in den
- Fig. 8 und 9: ein weiteres Mittel zur Arretierung in der Schließstellung.

In den Zeichnungen ist mit 1 eine Saugflasche bezeichnet, an die eine Leitung 2 anschließt, die aus den beiden Leitungsteilen 3 und 4 besteht. Diese beiden Leitungsteile 3 und 4 sollen durch eine Verbindungsvorrichtung 5 miteinander verbunden werden, wobei sichergestellt sein muß, daß diese Verbindung gasdicht ausgebildet ist.

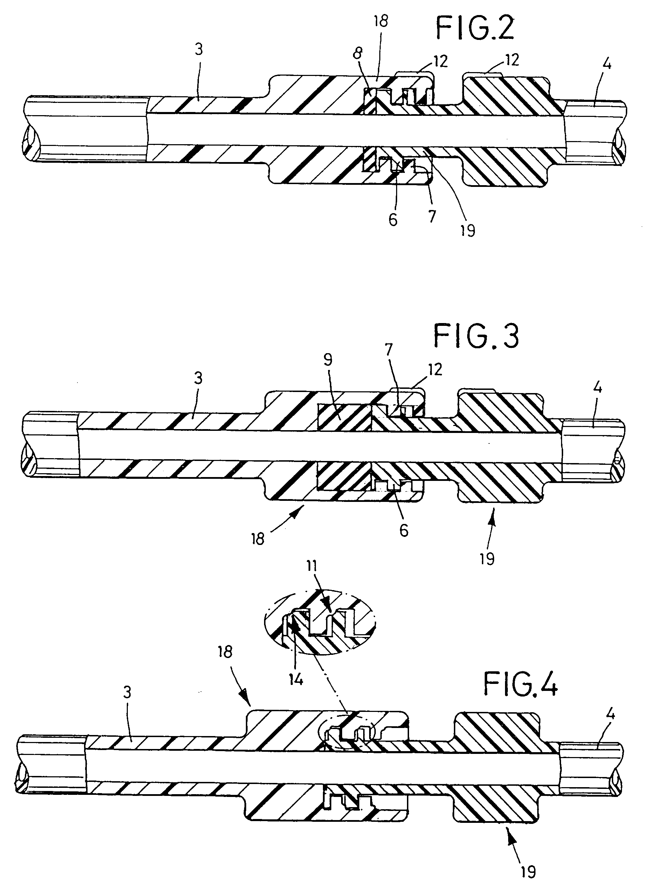

Als mögliche Ausführungsform ist in Fig. 2 eine aus einer Mutter 18 und einer Patrize 19 gebildete Verbindungsvorrichtung dargestellt, mit der die beiden Endteile der Leitungsteile 3 und 4 miteinander verbunden werden. Die Verbindung erfolgt über ein zusammenwirkendes Gewinde (Außengewinde 6 an der Patrize 19 und Innengewinde 7 an der Matrize 18), wobei zwischen den beiden zu verbindenden Teilen 18 und 19 ein Dichtring 8 aus gummielastischem Werkstoff angeordnet ist.

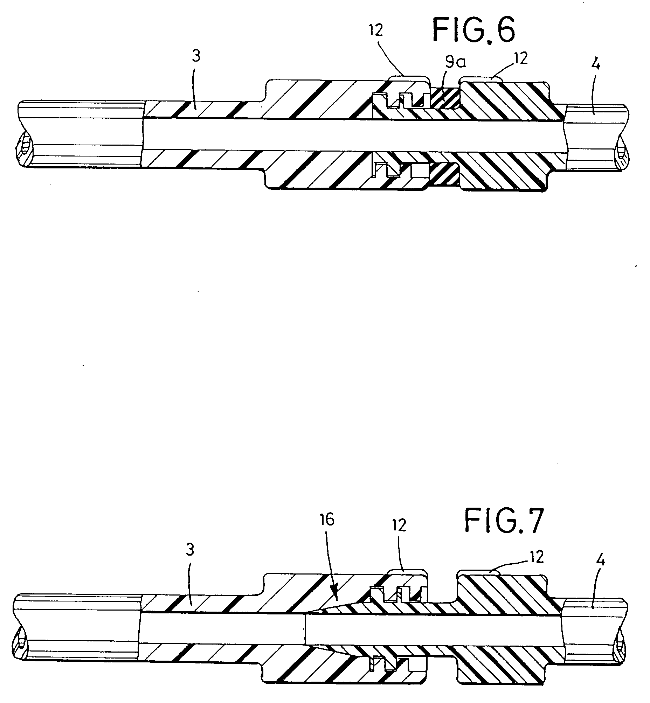

Bei der Anordnung gemäß Fig. 3 und Fig. 6 ist anstelle eines Dichtringes 8 eine Dichthülse 9 bzw. 9a vorgesehen, die ebenfalls aus elastischem, vorzugsweise gummielastischem Werkstoff besteht, und bei der Ausbildung gemäß Fig. 4 wird eine spezielle Gewindeart dargestellt, die so ausgebildet ist, daß das Gewinde freilaufend ist, aber bei einer vorgegebenen axialen Belastung, die durch das Verschrauben der beiden Gewindeteile herbeigeführt wird, die beiden Gewindebereiche miteinander in Kontakt kommen können, wobei an den Flanken des einen Gewindeteiles eine gewölbt ausgebildete Klemmfläche 11 ausgearbeitet ist, die bei Kontakt der beiden Gewindeteile mit dem Grat 14 des anderen Gewindeteiles in Kontakt kommt, wobei sich dieser Grat dann derart verformt, daß dadurch eine dichte Abdichtung zwischen den beiden durch das Gewinde verbundenen Leitungsteilen 3 und 4 erfolgt.

Um ein Überdrehen der Gewindeteile zu verhindern, sind Markierungen an den beiden Leitungsenden vorgesehen, die in den Zeichnungen ausführungshalber mit 12 bezeichnet sind, wobei bei fluchtender Stellung dieser Anzeigeteile 12 der Benutzer sicher sein kann, daß eine abdichtende Verbindung zwischen den beiden Leitungsenden hergestellt ist.

Bei der Ausführungsform gemäß Fig. 7 ist in den zusammenarbeitenden Patrizen- und Matrizenteilen 18 und 19 jeweils eine Konusfläche 16 vorgesehen, die beispielsweise durch mikroplastische Verformung die Abdichtung bewirkt. Zusätzlich könnte bei der Einrichtung gemäß Fig. 7 natürlich auch ein Dichtring 9a gemäß Fig. 6 eingebaut werden.

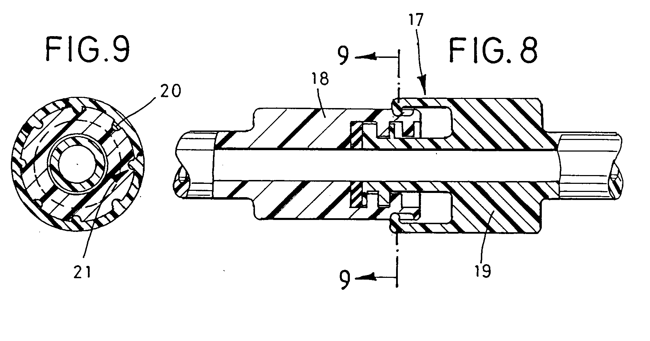

Bei der Ausführungsform gemaß den Fig. 8 und 9 weist das Patrizenteil 19 der Verbindung einen das Matrizenteil 18 übergreifenden Überwurf 17 auf. Der Überwurf 17 ist dabei mit nach innen vorstehenden Nocken 20 ausgerüstet, die in an der Oberseite des Matrizenteiles 18 vorgesehene Nuten eingreifen und dadurch die beiden Bauteile gegeneinander in einem gewissen Sinne verriegeln. Durch diese Anordnung wird erreicht, daß ein ungewolltes Lösen der Verbindung nicht möglich ist trotz der Rückstellkraft der innerhalb der Verbindung vorgesehenen elastischen Abdichtteile.

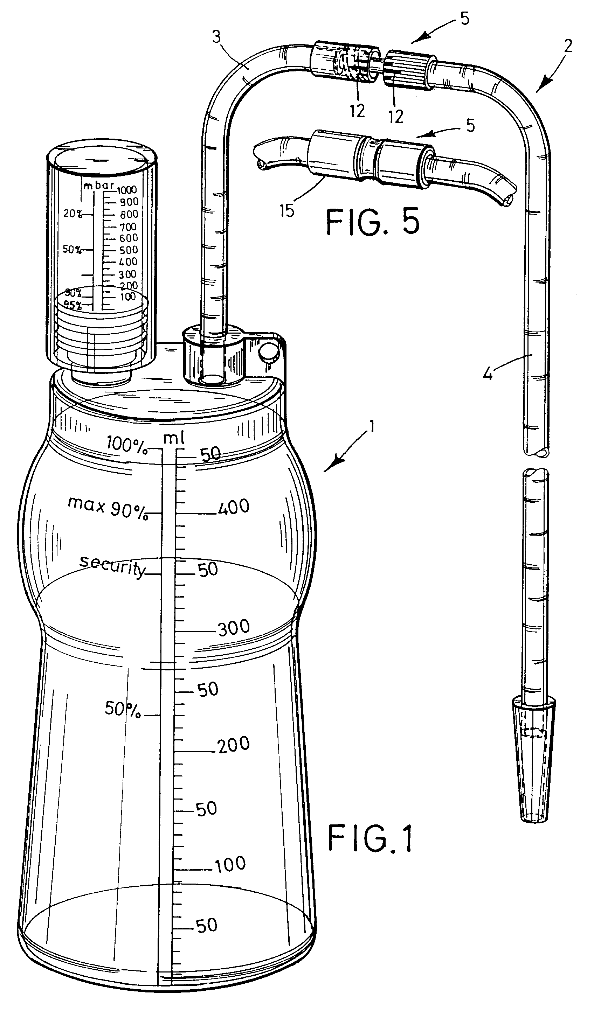

Bei der Anordnung gemäß Fig. 5 ist aus der Darstellung in Fig. 1 die Verbindungsvorrichtung 5 noch einmal herausgezeichnet und es ist ein elastischer Überzug 15 erkennbar, der die beiden Teile umgibt und der so eng anschließend über diese beiden Teile greift, daß dadurch ein ungewolltes Öffnen des Verschlusses entweder durch die in der Leitung herrschende Torsion oder ungewollter Weise durch einen Patienten nicht möglich ist.

Fig. 7 zeigt eine konische Aufnahme zur Verbesserung der Abdichtung.

## Patentansprüche

1. Verbindungsvorrichtung zum lösbaren Ankoppeln oder Entkoppeln einzelner Komponenten medizinischer, von einem Hohlraum durchsetzter Leitungen, gekennzeichnet durch eine, eine axiale Verschiebung der miteinander zu verbindenden Leitungsteile (3, 4) gegeneinander bewirkende Verbindungsvorrichtung (5), die die Verbindung oder Entkopplung bewirkt und ein Mittel zur Abdichtung des intraluminalen vom extraluminalen Raum enthält.

2. Verbindungsvorrichtung nach Anspruch 1, dadurch gekennzeichnet, daß die Verbindungsvorrichtung (5) durch zwei miteinander zusammenarbeitender Gewindebereiche (einem Außengewinde (6) und einem Innengewinde (7)) gebildet wird, wobei durch Verdrehen wenigstens eines Teiles (3 oder 4) eine Längsverschiebung des einen Leitungsteiles (3 oder 4) gegenüber dem anderen Leitungsteil (4 oder 3) auf das andere Leitungsteil zu erfolgt.

3. Vorrichtung nach Anspruch 2, dadurch gekennzeichnet, daß der Kontaktbereich im Gewinde mindestens 360^{o} beträgt.

4. Verbindungsvorrichtung nach 2, dadurch gekennzeichnet, daß der Kontaktbereich im Gewinde mindestens 540^{o} beträgt.

5. Verbindungsvorrichtung nach Anspruch 2, dadurch gekennzeichnet, daß der Kontaktbereich im Gewinde mindestens 720^{o} beträgt.

6. Vorrichtung nach einem der vorhergehenden Ansprüche, dadurch gekennzeichnet, daß die Verbindungsvorrichtung durch einen Bajonettverschluß gebildet ist.

7. Verbindungsvorrichtung nach einem der vorhhergehenden Ansprüche, dadurch gekennzeichnet, daß die die Verschiebung der beiden Leitungsteile (3, 4) bewirkende Vorrichtung mit einer Ausgestaltung (8, 9, 11, 9a, 16)) zur gasdichten Abdichtung der beiden Gewindebereiche gegeneinander ausgerüstet ist.

8. Vorrichtung nach einem der vorhergehenden Ansprüche, dadurch gekennzeichnet, daß die gasdichte Ausgestaltung ein Abdichtmittel (8, 9, 9a, 16)) umfaßt, das elastisch, vorzugsweise gummielastisch oder mikroplastisch verformbar ist.

9. Vorrichtung nach Anspruch 8, dadurch gekennzeichnet, daß das Abdichtmittel (8) aus einer ringförmigen Zwischenscheibe besteht.

10. Vorrichtung nach Anspruch 8, dadurch gekennzeichnet, daß das Abdichtmittel (9, 9a) aus einer Hülse besteht.

11. Vorrichtung nach Anspruch 8, dadurch gekennzeichnet, daß die gasdichte Ausgestaltung aus zwei zusammenwirkenden Konusflächen (16) besteht, die elastisch, vorzugsweise gummielastisch oder mikroplastisch verformbar sind.

12. Vorrichtung zur wirksamen Abdichtung zweier mittels Gewindeanschlüsse zu verbindender Hohlraumleitungen, dadurch gekennzeichnet, daß beide Leitungsenden mit Gewinde (6, 7) ausgerüstet sind, so daß beide Leitungsenden durch Verschrauben miteinander verbunden werden können, wobei das Gewinde (16) freilaufend ist, bis eine vorgegebene axiale Belastung auf die Gewindegänge einwirkt und einen positiven Kontakt zwischen den Gewinden (6 bzw. 7) ausübt, wobei in den Flanken wenigstens einiger Gewindegänge wenigstens eines Elementes eine schräg zur Gewindeachse verlaufende Klemmfläche (11) vorgesehen ist, die gewölbt ausgebildet ist und an der der Grat (14) des anderen Elementes des Gewindes bei Ausübung eines entsprechenden Anzuges sich anlegt.

13. Vorrichtung nach einem der vorhergehenden Ansprüche, dadurch gekennzeichnet, daß die Verbindungsvorrichtung (5) eine außen angeordnete Anzeige (12) aufweist.

14. Vorrichtung nach einem der vorhergehenden Ansprüche, gekennzeichnet durch eine Arretierungsvorrichtung, die das selbsttätige oder das ungewollte Lösen der Verbindung verhindert.

15. Vorrichtung nach einem der vorhergehenden Ansprüche, dadurch gekennzeichnet, daß der Druck auf die beiden zusammenwirkenden Abdichtbereiche mindestens 1 N/mm² beträgt.

16. Vorrichtung nach einem der vorhergehenden Ansprüche, dadurch gekennzeichnet, daß der Druck auf die beiden zusammenwirkenden Abdichtbereiche in der Größenordnung zwischen 1 und 10 N/mm² beträgt und vorzugsweise bei 5 N/mm² liegt. 5 N/mm² liegt.

17. Vorrichtung nach Anspruch 14, dadurch gekennzeichnet, daß die Arretierungsvorrichtung, um ein selbsttätiges oder ungewolltes Lösen der Verbindung zu verhindern, aus einem elastischen Überzug (15) besteht, der die beiden Teile der Verbindungsvorrichtung (5) umgibt.

18. Vorrichtung nach Anspruch 14, dadurch gekennzeichnet, daß die Arretierungsvorrichtung, die ein selbsttätiges oder ungewolltes Lösen der Verbindung verhindert, aus einem Überwurf (17) gebildet ist, der die Mutter (18) außen übergreift und mit vorstehenden Nocken (20) in an der Außenseite der Mutter vorgesehenen Nuten (21) eingreift.
